# FASCICULE DE BREVET EUROPEEN

(11) **EP 0 630 365 B1**
(45) Date de publication et mention de la délivrance du brevet: **22.10.1997**
(21) Numéro de dépôt: 93918746.4
(22) Date de dépôt: 08.03.1993
(51) Int. Cl.: C07C 227/18, C07C 229/34, C07D 205/08, C07D 305/14

(54) **PROCEDE DE PREPARATION DE LA BETA-PHENYLISOSERINE ET DE SES ANALOGUES**
PROZESS ZUR HERSTELLUNG VON BETA-PHENYLISOSERIN UND ANALOGEN VERBINDUNGEN
METHOD FOR PREPARING BETA-PHENYLISOSERINE AND ANALOGUES THEREOF

(30) Priorité: 10.03.1992 FR 9202821
(43) Date de publication de la demande: 28.12.1994
(73) Titulaire: RHONE-POULENC RORER S.A., 92160 Antony (FR)
(72) Inventeur: BOURZAT, Jean-Dominique, F-94300 Vincennes (FR); COMMERCON, Alain, F-94400 Vitry-sur-Seine (FR)
(74) Mandataire: Pilard, Jacques
(86) Numéro de dépôt international: FR9300224
(87) Numéro de publication internationale: WO9317997

(56) Documents cités:
- EP-A- 0 400 971
- EP-A- 0 402 322
- WO-A-91/13053
- JOURNAL OF ORGANIC CHEMISTRY, vol. 56, no. 18, 30 août 1991, Easton, US, pages 5263-5277, I. OJIMA et al, "Azetidines and Bisazetidines. Their Synthesis and Use as the Key Intermediates to Enantiomerically Pure Diamines, Amino Alcohols, and Polyamines"
- TETRAHEDRON LETTERS, vol. 31, no. 44, 1990, Oxford, GB, pages 6429 - 6432, C. PALOMO et al, "Highly Stereoselective Synthesis of alpha-Hydroxy beta-Amino acids through beta-Lactams: application to the Synthesis of the Taxol and Bestatin Side Chains and Related Systems"

## Description

La présente invention concerne un nouveau procédé de préparation de la β-phénylisosérine et de ses analogues de formule générale : 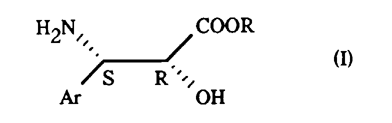 qui sont particulièrement utiles pour préparer les dérivés du taxane qui présentent des activités antitumorales et antileucémiques remarquables.

Dans la formule générale (I), Ar représente un radical aryle et R représente un atome d'hydrogène ou un radical alcoyle contenant 1 à 4 atomes de carbone, éventuellement substitué par un radical phényle, ou un radical phényle.

De préférence, Ar représente un radical phényle ou α- ou β-naphtyle éventuellement substitué par un ou plusieurs atomes ou radicaux choisis parmi les atomes d'halogène (fluor, chlore, brome, iode) et les radicaux alcoyles, aryles, aralcoyles, alcoxy, alcoylthio, aryloxy, arylthio, hydroxy, hydroxyalcoyle, mercapto, formyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone et que les radicaux aryles sont des radicaux phényles ou α- ou β-naphtyles.

Plus particulièrement, Ar représente un radical phényle éventuellement substitué par un ou plusieurs atomes ou radicaux, identiques ou différents, choisis parmi les atomes d'halogène et les radicaux alcoyles, alcoxy, amino, alcoylamino, dialcoylamino, acylamino, alcoxycarbonylamino et trifluorométhyle.

Plus particulièrement encore, Ar représente un radical phényle éventuellement substitué par un atome de chlore ou de fluor ou par un radical alcoyle (méthyle), alcoxy (méthoxy), dialcoylamino (diméthylamino), ou acylamino (acétylamino).

Il est connu de préparer la β-phénylisosérine par hydrolyse d'un lactame dans les conditions décrites par C. Palomo et coll., Tetrahedron Letters, 31, 6429-6439 (1990).

La thréo-β-phénylisosérine peut être obtenue par action de l'ammoniac sur un ester de l'acide cis-β-phénylglycidique suivie de l'action de la barite, afin d'éviter la racémisation sur la β-phénylisosérine-amide intermédiairement obtenue dans les conditions décrites par E. Kamandi et coll., Arch. Pharmaj., 307 871-878 (1974).

La β-phénylisosérine peut être également obtenue dans les conditions décrites dans la demande internationale PCT WO-A-91/13053 en passant intermédiairement par la N-benzyl-β-phénylisosérine.

Selon la présente invention, les produits de formule générale (I) sont obtenus par hydrogénolyse d'un produit de formule générale : 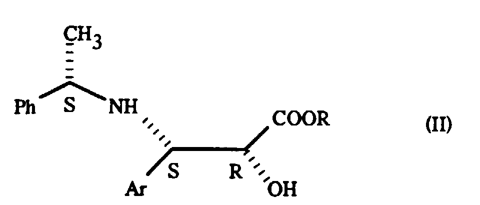 dans laquelle Ar et R sont définis comme précédemment et Ph représente un radical phényle éventuellement substitué par un ou plusieurs atomes ou radicaux choisis parmi les atomes d'halogène (fluor, chlore, brome, iode) et les radicaux alcoxy contenant 1 à 4 atomes de carbone, alcoylthio contenant 1 à 4 atomes de carbone, amno, alcoylamino contenant 1 à 4 atomes de carbone, dialcoylamino dont chaque partie alcoyle contient 1 à 4 atomes de carbone ou nitro. Plus particulièrement, Ph représente un radical phényle éventuellement substitué par un ou plsieurs radicaux identiques ou différents choisis parmi les radicaux méthoxy, méthylthio, méthylamino, diméthylamino ou nitro.

Généralement, l'hydrogénolyse est effectuée au moyen d'hydrogène en présence de catalyseur.

Plus particulièrement, on utilise comme catalyseur un palladium sur charbon contenant 1 à 10 % en poids de palladium ou le dihydroxyde de palladium sur charbon contenant jusqu'à 10% en poids de palladium.

L'hydrogénolyse est effectuée dans un solvant organique ou un mélange de solvants organiques.

Il est particulièrement avantageux d'opérer dans l'acide acétique éventuellement associé à un alcool aliphatique contenant 1 à 4 atomes de carbone. D'un intérêt tout particulier est un mélange d'acide acétique et de méthanol.

Selon un mode préféré de mise en oeuvre du procédé, on opère sous une pression d'hydrogène qui peut être comprise entre 1 et 50 bars.

La température de mise en oeuvre du procédé est généralement comprise entre 20 et 80°C et de préférence entre 50 et 70°C.

L'hydrogène nécessaire à l'hydrogénolyse peut aussi être fourni par un composé qui libère de l'hydrogène par réaction chimique ou par décomposition thermique tel que le formiate d'ammonium.

Le produit de formule générale (II) peut être obtenu par hydrolyse ou alcoolyse d'un produit de formule générale : 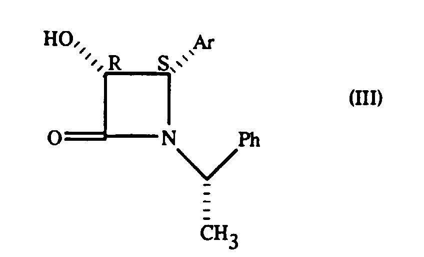 dans laquelle Ar et Ph sont définis comme précédemment.

Il est particulièrement avantageux d'effectuer une alcoolyse au moyen d'un alcool de formule générale R-OH dans laquelle R est défini comme précédemment en opérant en milieu acide.

De préférence, on effectue l'alcoolyse au moyen de méthanol en présence d'un acide minéral fort tel que l'acide chlorhydrique.

Il est avantageux d'effectuer l'alcoolyse à une température voisine de la température de reflux du mélange réactionnel.

Le produit de formule générale (III) peut être obtenu par saponification ou hydrogénolyse d'un produit de formule générale : 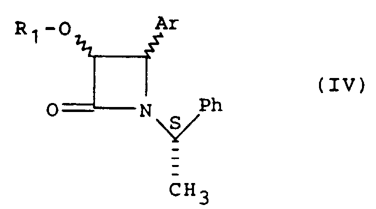 dans laquelle Ar et Ph sont définis comme précédemment et R₁ représente un groupement protégeant la fonction alcool sous forme d'ester ou d'ether, suivie de la séparation du diastéréoisomère 3R,4S de formule générale (III) des autres diastéréoisomères.

Plus particulièrement R₁ représente un radical alcoyle, phénylalcoyle ou phényle ou un radical R'₁-CO dans lequel R'₁ représente un radical alcoyle, phénylalcoyle ou phényle.

Généralement, lorsque la fonction alcool est protégée sous forme d'ester, on effectue une saponification au moyen d'une base minérale ou organique telle que l'ammoniaque, la lithine, la soude ou la potasse dans un solvant convenable.

Comme solvant, on utilise de préférence un milieu hydroorganique tel qu'un mélange méthanol-eau ou tétrahydrofuranne-eau. La réaction est mise en oeuvre à une température comprise entre -10 et +20°C.

Généralement, lorsque la fonction alcool est protégée sous forme d'éther, on effectue une hydrogénolyse au moyen d'hydrogène, éventuellement généré in situ, par exemple, par décomposition du formiate d'ammonium, en présence d'un catalyseur tel que le palladium sur noir contenant de 1 à 10% de palladium (p/p).

La présente invention concerne également les produits de formule (IV) pour lesquels Ar représente un radical phényle ou α- ou β-naphtyle éventuellement substitué par un ou plusieurs atomes ou radicaux choisis parmi les atomes d'halogène (fluor, chlore, brome, iode) et les radicaux alcoyles, aryles, aralcoyles, alcoxy, alcoylthio, aryloxy, arylthio, hydroxy, hydroxyalcoyle, mercapto, formyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone et que les radicaux aryles sont des radicaux phényles ou α- ou β-naphtyles, Ph représente un radical phényle éventuellement substitué par un ou plusieurs atomes ou radicaux choisis parmi les atomes d'halogène et les radicaux alcoxy contenant 1 à 4 atomes de carbone, alcoylthio contenant 1 à 4 atomes de carbone, amno, alcoylamino contenant 1 à 4 atomes de carbone, dialcoylamino dont chaque partie alcoyle contient 1 à 4 atomes de carbone ou nitro et R₁ représente un radical alcoyle, phénylalcoyle ou phényle ou un radical R'₁-CO dans lequel R'₁ représente un radical alcoyle, phénylalcoyle ou phényle, étant entendu que, lorsque Ar représente un radical phényle, Ph est différent d'un radical phényle non substitué.

La séparation du diastéréoisomère (3R,4S) peut être effectuée par cristallisation sélective dans un solvant organique convenable tel que l'acétate d'éthyle éventuellement en présence d'un hydrocarbure aliphatique tel que l'hexane ou par chromatographie sur silice.

Le produit de formule générale (IV) peut être obtenu par cycloaddition d'une imine de formule générale : 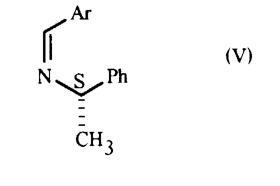 dans laquelle Ar et Ph sont définis comme précédemment, sur un halogénure d'acide de formule générale : 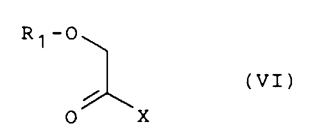 dans laquelle R₁ est défini comme précédemment et X représente un atome d'halogène tel qu'un atome de brome ou de chlore.

Généralement la réaction est effectuée à une température comprise entre -20 et 50°C, de préférence au voisinage de 0°C en présence d'une base choisie parmi les amines tertiaires (triéthylamine, N-méthyl-morpholine) ou la pyridine dans un solvant organique choisi parmi les hydrocarbures aliphatiques éventuellement halogénés tels que le chlorure de méthylène ou le chloroforme et les hydrocarbures aromatiques tels que le benzène, le toluène ou les xylènes.

Le produit de formule générale (V) peut être obtenu dans les conditions décrites par M. Furukawa et coll., Chem. Pharm. Bull., 25(1), 181-184 (1977).

Le produit de formule générale (I) dans laquelle R représente un atome d'hydrogène peut aussi être obtenu par saponification d'un produit de formule générale (I) dans laquelle R représente un radical alcoyle contenant 1 à 4 atomes de carbone, éventuellement substitué par un radical phényle ou un radical phényle.

Généralement la saponification est réalisée au moyen d'une base minérale telle qu'un hydroxyde de métal alcalin (lithine, soude), un carbonate ou un bicarbonate alcalin (bicarbonate de sodium, carbonate de potassium) en milieu hydroalcoolique tel qu'un mélange méthanol-eau en opérant à une température comprise entre 10 et 40°C, de préférence voisine de 25°C.

Les acides de formule générale (I) sont particulièrement utiles pour préparer les dérivés du taxane de formule générale : 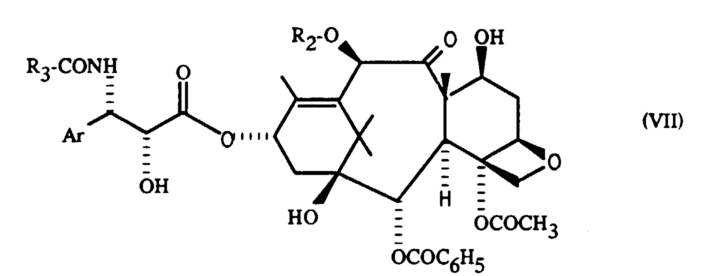 dans laquelle Ar est défini comme précédemment, R₂ représente un atome d'hydrogène ou un radical acétyle et R₃ représente un radical phényle éventuellement substitué par un ou plusieurs atomes ou radicaux, identiques ou différents, choisis parmi les atomes d'halogène et les radicaux alcoyles, hydroxy, alcoxy, alcanoyle, alcanoyloxy, nitro, amino, alcoylamino, dialcoylamino, carbamoyle ou trifluorométhyle, les radicaux alcoyles et les portions alcoyles des autres radicaux contenant 1 à 4 atomes de carbone, ou bien R₃ représente un radical R₄-O- dans lequel R₄ représente :
- un radical alcoyle droit ou ramifié contenant 1 à 8 atomes de carbone, alcényle contenant 3 à 6 atomes de carbone, cycloalcoyle contenant 3 à 6 atomes de carbone, cycloalcényle contenant 4 à 6 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 4 à 6 atomes de carbone, alcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitué par un ou plusieurs atomes ou radicaux choisis parmi les atomes d'halogène et les radicaux alcoyles contenant 1 à 4 atomes de carbone ou alcoyloxy contenant 1 à 4 atomes de carbone,
- ou un radical hétérocyclyle azoté saturé ou non saturé contenant 5 ou 6 chaînons et éventuellement substitué par un ou plusieurs radicaux alcoyles contenant 1 à 4 atomes de carbone,
qui présentent des propriétés antitumorales et antileucémiques remarquables.

Le produit de formule générale (VII) dans laquelle Ar représente un radical phényle, R₂ représente un radical acétyle et R₃ représente un radical phényle est connu sous le nom de taxol et celui pour lequel Ar représente un radical phényle, R₂ représente un atome d'hydrogène et R₃ représente un radical tert.butoxy est connu sous le nom de Taxotère.

Les dérivés du taxane de formule générale (VII) peuvent être obtenus par action d'un acide de formule générale : 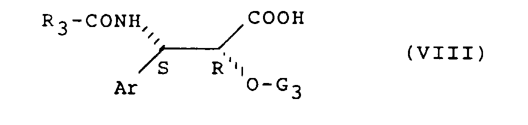 dans laquelle Ar et R₃ sont définis comme précédemment et G₃ représente un groupement protecteur de la fonction hydroxy tel qu'un radical méthoxyméthyle, (éthoxy-1) éthyle, benzyloxyméthyle, (β-triméthylsilyléthoxy) méthyle, tétrahydropyrannyle, trichloro-2,2,2 éthoxyméthyle ou trichloro-2,2,2 éthoxycarbonyle, éventuellement sous forme d'halogénure, d'anhydride ou d'anhydride mixte, sur un dérivé du taxane de formule générale : 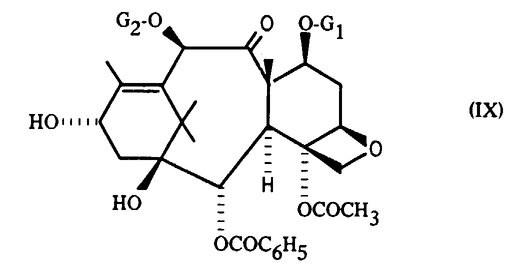 dans laquelle G₁ représente un groupement protecteur de la fonction hydroxy tel qu'un radical trichloro-2,2,2 éthoxycarbonyle ou trialcoylsilyle, dialcoylarylsilyle, alcoyldiarylsilyle ou triarylsilyle dans lequel chaque partie alcoyle contient 1 à 4 atomes de carbone et chaque partie aryle représente de préférence un radical phényle et G₂ représente un radical acétyle ou un groupement protecteur de la fonction hydroxy tel qu'un radical trichloro-2,2,2 éthoxycarbonyle pour donner un produit de formule générale : 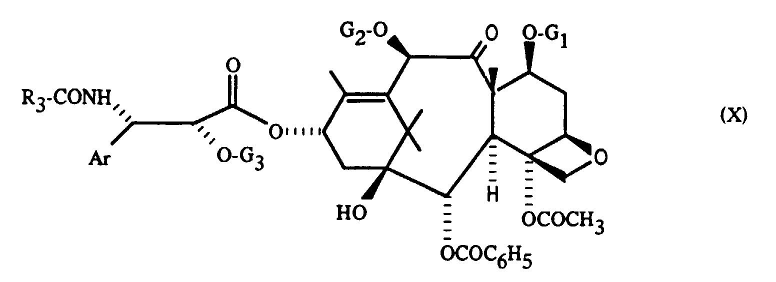 dans laquelle Ar, R₃, G₁, G₂ et G₃ sont définis comme précédemment, suivie du remplacement des groupements G₁, G₂ et G₃ par des atomes d'hydrogène.

Généralement l'estérification est effectuée en présence d'un agent de condensation tel qu'un carbodiimide comme le dicyclohexylcarbodiimide ou un carbonate réactif comme le pyridyl-2 carbonate et d'un agent d'activation tel qu'une aminopyridine comme la diméthylamino-4 pyridine ou la pyrrolidino-4 pyridine en opérant dans un solvant organique tel qu'un hydrocarbure aromatique (benzène, toluène, xylène, éthylbenzène, isopropylbenzène, chlorobenzène), un éther (tétrahydrofuranne), un nitrile (acétonitrile) ou un ester (acétate d'éthyle) à une température comprise entre 0 et 90°C.

Le remplacement des groupements protecteurs G₁, G₂ et G₃ par des atomes d'hydrogène est effectué généralement par traitement par le zinc en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone en présence de zinc lorsque l'un des groupements protecteurs représente un radical trichloro-2,2,2 éthoxycarbonyle ou par traitement en milieu acide lorsque l'un des groupements protecteurs représente un radical silylé.

L'acide de formule générale (VIII) peut être obtenu par saponification d'un ester de formule générale : 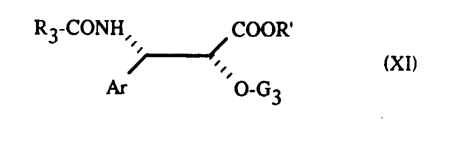 dans laquelle Ar, R₃ et G₃ sont définis comme précédemment et R' représente un radical alcoyle contenant 1 à 4 atomes de carbone, éventuellement substitué par un radical phényle, ou un radical phényle, au moyen d'une base minérale telle qu'un hydroxyde de métal alcalin (lithine, soude), un carbonate ou un bicarbonate de métal alcalin (bicarbonate de sodium, carbonate de potassium) en milieu hydroalcoolique tel qu'un mélange méthanol-eau en opérant à une température comprise entre 10 et 40°C, de préférence voisine de 25°C.

Le produit de formule générale (XI) peut être obtenu dans les conditions habituelles de préparation des éthers, et plus particulièrement selon les procédés décrits par J-N. Denis et coll., J. Org. Chem., 51, 46-50 (1986) à partir du produit de formule générale : 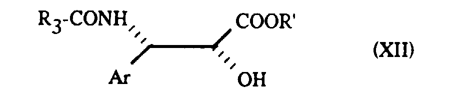 dans laquelle Ar, R₃ et R' sont définis comme précédemment.

Le produit de formule générale (XII) peut être obtenu par action d'un halogénure de benzoyle dont le noyau phényle peut être éventuellement substitué ou par action d'un produit de formule générale :

R₄-O-CO-X (XIII)

dans laquelle R₄ est défini comme précédemment et X représente un atome d'halogène (fluor, chlore) ou un reste -O-R₄ ou -O-CO-OR₄, sur un produit de formule générale (I) dans laquelle R représente un radical alcoyle contenant 1 à 4 atomes de carbone, éventuellement substitué par un radical phényle, ou un radical phényle.

Généralement, on opère dans un solvant organique tel que le chlorure de méthylène en présence d'une base minérale telle que le bicarbonate de sodium.

Le produit de formule générale (XII) dans laquelle Ar représente un radical phényle substitué par un radical cyano peut être obtenu par deshydratation d'un produit de formule générale (XII) dans laquelle Ar représente un radical phényle substitué par un radical carbamoyle et la fonction alcool est protégée de préférence par un radical silylé, suivi du remplacement du groupement protecteur par un atome d'hydrogène.

La deshydrataion peut être généralement effectuée selon les méthodes habituelles de préparation des nitriles à partir des amides. Par exemple, on utilise l'oxychlorure de phosphore dans la pyridine.

Les dérivés du taxane de formule générale (VII) peuvent aussi être obtenus en transformant d'abord le produit de formule générale (XII) en dérivé de l'oxazolidine de formule générale : 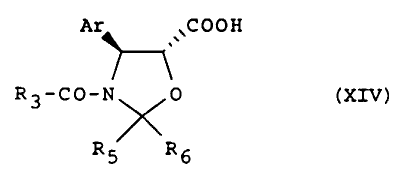 dans laquelle Ar et R₃ sont définis comme précédemment et R₅ et R₆, identiques ou différents, représentent un atome d'hydrogène ou un radical alcoyle contenant 1 à 4 atomes de carbone ou un radical aryle de préférence un radical phényle éventuellement substitué par un ou plusieurs radicaux alcoxy contenant 1 à 4 atomes de carbone, ou bien R₅ représente un radical alcoxy contenant 1 à 4 atomes de carbone ou un radical trihalométhyle tel que trichlorométhyle et R₆ représente un atome d'hydrogène, ou bien R₅ et R₆ forment ensemble avec l'atome de carbone auquel ils sont liés un cycle ayant 4 à 7 chaînons, puis en estérifiant le dérivé du taxane de formule générale (IX) au moyen de l'acide de formule générale (XIV) pour obtenir un produit de formule générale : 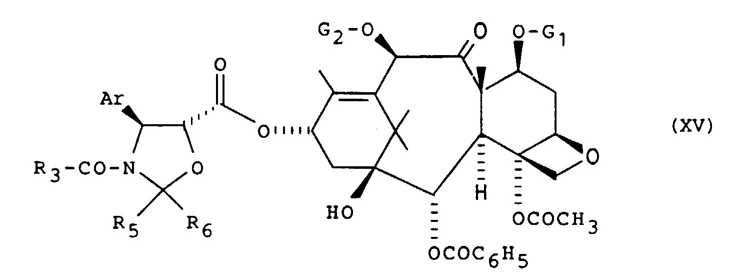 dans laquelle Ar, G₁, G₂, R₃, R₅ et R₆ sont définis comme précédemment, qui est transformé en dérivé du taxane de formule générale (VII) en passant intermédiairement, lorsque R₅ et R₆, identiques ou différents, représentent un radical alcoyle contenant 1 à 4 atomes de carbone, ou un radical aryle de préférence un radical phényle éventuellement substitué, ou bien R₅ représente un radical trihalométhyle ou un radical phényle substitué par un radical trihalométhyle et R₆ représente un atome d'hydrogène, ou bien R₅ et R₆ forment ensemble avec l'atome de carbone auquel ils sont liés un cycle ayant 4 à 7 chaînons, par un dérivé du taxane de formule générale : 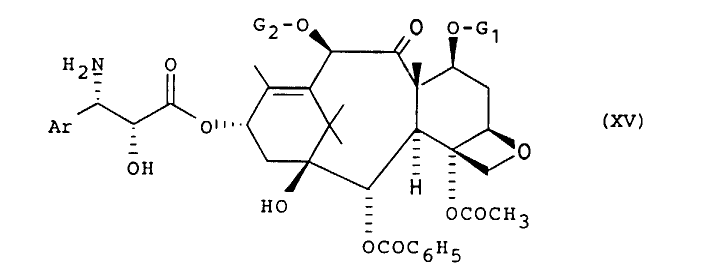 qui est acylé au moyen de chlorure de benzoyle ou d'un produit de formule générale (XIII) en opérant par exemple dans les conditions décrites dans la demande PCT WO 9209589, avant d'obtenir un produit de formule générale : 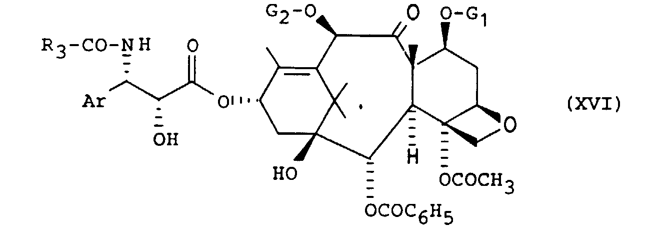 dont les groupements protecteurs G₁ et G₂ sont remplacés par des atomes d'hydrogène dans les conditions décrites précédemment.

Les exemples suivants illustrent l'invention.

### EXEMPLE 1

A 0,91 g d'une dispersion à 3 % de palladium dans du charbon activé en poudre on ajoute une solution de 1,6 g d'hydroxy-2 [phényl-1-(S)] éthylamino-3 phényl-3 propionate-(2R,3S) de méthyle dans un mélange de 30 cm3 de méthanol et de 10 cm3 d'acide acétique. Le mélange réactionnel est chauffé à une température de 65°C pendant 4 heures, sous agitation et sous une pression de 2600 kPa (26 bars) d'hydrogène, dans un autoclave de 250 cm3 en acier inoxydable. Le mélange réactionnel est ensuite refroidi à une température voisine de 20°C et filtré sur verre fritté garni de célite. Le verre fritté est lavé par 3 fois 10 cm3 de méthanol et les filtrats sont réunis puis concentrés à sec sous pression réduite (2,7 kPa) à une température voisine de 40°C. Le résidu est additionné de 40 cm3 d'eau distillée et la solution obtenue est alcalinisée à un pH voisin de 7 par addition de 8 cm3 d'une solution aqueuse 7,5N d'hydroxyde de sodium puis extraite par 4 fois 60 cm3 de dichlorométhane. Les phases organiques sont réunies, séchées sur sulfate de magnésium, filtrées puis concentrées à sec sous pression réduite (2,7 kPa) à 40°C. On obtient ainsi 0,74 g de cristaux blancs que l'on recristallise dans 10 cm3 d'un mélange d'oxyde de diisopropyle et d'acétate d'éthyle (70-30 en volumes) pour donner 0,54 g d'amino-3 hydroxy-2 phényl-3 propionate-(2R,3S) de méthyle sous forme de cristaux blancs fondants à 101°C et dont les caractéristiques sont les suivantes :
- pouvoir rotatoire : [α]²⁰_{D} = -19° (c = 0,51 ; méthanol)
- spectre de RMN (300 MHz ; CDCl₃)
   δ (ppm) : 2,22 (m, 3H : -NH₂ et OH) ; 3,81 (s, 3H : -COOCH₃); 4,32 (s, 2H : -CHOH et -CHNH₂); 7,20 à 7,5 (m, 5H : -C₆H₅).

L'hydroxy-2 [phényl-1-(S)] éthylamino-3 phényl-3 propionate-(2R,3S) de méthyle peut être préparé de la manière suivante :

Une solution de 0,8 g d'hydroxy-3 phényl-4 [phényl-1-(S)] éthyl-1 azétidinone-2-(3R,4S) dans un mélange de 30 cm3 de méthanol et de 6 cm3 d'une solution aqueuse 6N d'acide chlorhydrique est chauffée au reflux (65°C) pendant 20 heures, puis refroidie à une température voisine de 20°C et concentrée à sec sous pression réduite (2,7 kPa) à une température voisine de 40°C. Le résidu est additionné de 20 cm3 d'eau distillée et alcalinisé jusqu'à un pH voisin de 7 par addition d'une solution aqueuse 7,5N d'hydroxyde de sodium puis extrait par 3 fois 25 cm3 de dichlorométhane. Les phases organiques sont réunies, séchées sur sulfate de magnésium, filtrées puis concentrées à sec sous pression réduite (2,7 kPa) à 40°C. On obtient ainsi 0,74 g d'hydroxy-2 [(phényl-1(S)] éthylamino-3 phényl-3 propionate-(2R,3S) de méthyle sous forme d'une huile jaune pâle dont les caractéristiques sont les suivantes :
- pouvoir rotatoire : [α]²⁰_{D} = -22,7° (c = 1,00 ; méthanol)
- spectre de RMN (200 MHz ; CDCl₃)
   δ (ppm) : 1,34 (d, 3H, J = 7Hz : -CCH₃) ; 2,7 (m, 2H : -CNHC- et -OH) ; 3,71 (q, 1H, J = 7 Hz : -CHNH-); 3,84 (s, 3H : -COOCH₃); 4,2 (d, 1H, J = 4 Hz : -CHOH-) ; 4,35 (d, 1H, J = 4 Hz ; -CHNH-) ; 7,20 à 7,45 (m, 5H : -C₆H₅).

L'hydroxy-3 phényl-4 [phényl-1-(S)] éthyl-1 azétidone-2(3R,4S) peut être préparée selon l'une des méthodes suivantes :

1) A un mélange de 120 cm3 d'une solution aqueuse 1N d'hydroxyde de potassium et de 90 cm3 de tétrahydrofuranne on ajoute en 35 minutes, sous agitation et à une température voisine de 0°C, une solution de 3,3 g d'un mélange en proportion molaire 75/25 des deux diastéréoisomères d'acétoxy-3 phényl-4 [phényl-1-(S)] éthyl-1 azétidinone-2 forme A et forme B dans 120 cm3 de tétrahydrofuranne. L'addition terminée le milieu réactionnel est agité à une température voisine de 0°C pendant 1 heure puis additionné de 120 cm3 d'une solution aqueuse saturée d'hydrogénocarbonate de sodium et de 100 cm3 d'eau distillée. La phase aqueuse est séparée par décantation et réextraite par 3 fois 100 cm3 d'acétate d'éthyle. Les phases organiques sont réunies, séchées sur sulfate de magnésium, filtrées puis concentrées à sec sous pression réduite (2,7 kPa) à 40°C. On obtient ainsi 2,8 g de cristaux blancs que l'on recristallise dans 35 cm3 d'un mélange d'acétate d'éthyle et d'hexane (80-20 en volumes) pour donner 1,92 g d'hydroxy-3 phényl-4 [phényl-1-(S)] éthyl-1 azétidinone-2-(3R,4S) sous forme de cristaux blancs fondant à 162°C et dont les caractéristiques sont les suivantes :
- pouvoir rotatoire : [α]²⁰_{D} = +132° (c = 1,08 ; méthanol)
- spectre de RMN (200 MHz ; CDCl₃)
   δ (ppm) : 1,41 (d, 3H, J = 7 Hz : -CHCH₃) ; 2,36 (d, 1H, J = 8,5 Hz : -OH) ; 4,58 (d, 1H, J = 4,5 Hz : -CHC₆H₅) ; 4,90 (dd, 1H, J = 8,5 Hz et 4,5 Hz : -CHOH-) ; 5,06 (q, 1H, J = 7 Hz : -CHCH₃) ; 7,20 à 7,50 (m, 5H : -C₆H₅).

Le mélange de la forme A et de la forme B de l'acétoxy-3 phényl-4 [phényl-1-(S)] éthyl-1 azétidone-2 peut être préparé de la manière suivante :

A une solution de 14,63 g de N-benzylidène-(phényl-1 éthylamine)-(S) dans 180 cm3 de chloroforme on ajoute, sous agitation et à une température voisine de 20°C, 19,6 cm3 de triéthylamine puis on refroidit le mélange réactionnel jusqu'à une température voisine de -20°C et additionne goutte à goutte, en 75 minutes et en se maintenant à cette température, 5,17 cm3 de chlorure d'acétoxy-2 acétyle dans 90 cm3 de chloroforme. La solution obtenue est agitée pendant 16 heures à une température voisine de 20°C puis additionnée de 300 cm3 d'une solution aqueuse 2,7N d'acide chlorhydrique. La phase organique est séparée par décantation, lavée par 2 fois 300 cm3 d'eau distillée puis par 300 cm3 d'une solution aqueuse saturée d'hydrogénocarbonate de sodium, séchée sur sulfate de magnésium, filtrée puis concentrée à sec sous pression réduite (2,7 kPa) à 40°C. On obtient ainsi 16,5 g d'une huile brune que l'on purifie par chromatographie sur 800 g de silice (0,04-0,063 mm) contenus dans une colonne de 6,8 cm de diamètre [éluant : cyclohexane-acétate d'éthyle (70-30 en volumes)] en recueillant des fractions de 22 cm3. Les fractions 100 à 153 sont réunies et concentrées à sec sous pression réduite (0,27 kPa) à 40°C. On obtient ainsi 10,65 g d'un mélange en proportion molaire 75/25 de deux diastéréoisomères d'acétoxy-3 phényl-4 [phényl-1-(S)] éthyl-1 azétidinone-2 sous forme d'une huile jaune.

Le N-benzylidène-(phényl-1 éthylamine)-(S) peut être préparé selon la méthode décrite par M. Furukawa et coll., Chem. Pharm. Bull., 1977, 25(1), 181-184.

2) En opérant comme précédemment, mais à partir de 100 mg d'un mélange en proportion molaire 70/30 des deux diastéréoisomères d'isobutyryloxy-3 phényl-4 [phényl-1-(S)]éthyl-1 azétidinone-2 forme A et forme B, on obtient 82 mg d'hydroxy-3 phényl-4 [phényl-1-(S)]éthyl-1 azétidinone-2-(3R,4S) sous forme de cristaux blancs fondants à 162°C dont les caractéristiques physiques sont identiques à celles du produit obtenu précédemment.

Le mélange des formes A et B de l'isobutyryloxy-3 phényl-4 [phényl-1-(S)]éthyl-1 azétidinone-2 peut être préparé en opérant comme précédemment mais à partir de 1,91 g de N-benzylidène-(phényl-1 éthylamine)-(S) et de 1 g de chlorure d'isobutyryloxy-2 acétyle. On obtient ainsi 1,27 g d'un mélange en proportion molaire : 70/30 de deux diastéréoisomères d'isobutyryloxy-3 phényl-4 [phényl-1-(S)]éthyl-1 azétidinone-2 sous forme d'une huile jaune.

Le chlorure d'isobutyryloxy-2 acétyle peut être préparé de la manière suivante :

A une solution de 5 g d'acide glycolique dans 100 cm3 de dichlorométhane, maintenue sous atmosphère d'argon, on ajoute sous agitation et à une température voisine de 20°C 18,3 cm3 de triéthylamine puis on refroidit le mélange réactionnel jusqu'à une température voisine de 5°C et additionne goutte à goutte, en 30 minutes et en se maintenant à cette température, 13,8 cm3 de chlorure d'isobutyryle. La solution obtenue est agitée pendant 3 heures à une température voisine de 20°C. Le précipité apparu est séparé par filtration et lavé par 2 fois 10 cm3 de dichlorométhane. Les filtrats réunis sont lavés par 60 cm3 d'une solution aqueuse saturée de chlorure d'ammonium puis par 30 cm3 d'une solution aqueuse saturée de chlorure de sodium, séchés sur sulfate de magnésium, filtrés puis concentrés à sec sous pression réduite (2,7 kPa) à 40°C. On obtient ainsi 13 g d'une huile jaune à laquelle on ajoute 24 cm3 de chlorure de sulfinyle. La solution obtenue est chauffée au reflux pendant 2,5 heures puis distillée sous pression réduite (0,07 kPa ; 0,5 mmHg). On obtient ainsi 3,4 g de chlorure d'isobutyryloxy-2 acétyle sous forme d'un liquide incolore distillant à 45-50°C sous une pression de 0,07 kPa.

3) A 43 mg d'une dispersion à 10 % de palladium dans du charbon en poudre, on ajoute une solution de 91 mg d'un mélange en proportion molaire 60/40 des deux diastéréoisomères de benzyloxy-3 phényl-4 [phényl-1-(S)]éthyl-1 azétidinone-2 forme A et forme B dans 6 cm3 de méthanol puis 32 mg de formiate d'ammonium. Le mélange réactionnel est maintenu sous agitation et sous atmosphère d'argon pendant 72 heures à une température voisine de 20°C puis on ajoute 56 mg de la dispersion de palladium à 10 % et 128 mg de formiate d'ammonium. Le mélange réactionnel est maintenu sous agitation à la même température pendant 26 heures. Le mélange réactionnel est ensuite filtré sur verre fritté garni de célite. Le verre fritté est lavé par 3 fois 5 cm3 de dichlorométhane puis les filtrats réunis sont concentrés sous pression réduite (2,7 kPa) à une température voisine de 40°C. On obtient ainsi 70 mg de cristaux blancs que l'on purifie par chromatographie sur gel de silice déposé sur plaque (gel de 1 mm d'épaisseur ; plaque de 20x20 cm) par fractions de 10 mg. Après localistion aux rayons U.V. de la zone correspondant au produit cherché, cette zone est grattée et la silice est recueillie puis lavée sur verre fritté par 10 fois 5 cm3 de dichlorométhane et par 5 fois 2 cm3 de méthanol. les filtrats sont réunis et concentrés à sec sous pression réduite (0,27 kPa) à 40°C. On obtient ainsi 28 mg d'hydroxy-3 phényl-4 [phényl-1-(S)]éthyl-1 azétidinone-2-(3R,4S) sous forme de cristaux blancs fondants à 162°C dont les caractéristiques physiques sont identiques à celles du produit obtenu précédemment.

Le mélange des formes A et B du benzyloxy-3 phényl-4 [phényl-1-(S)]éthyl-1 azétidinone-2 peut être préparé en opérant comme précédemment mais à partir de 2,0 g de N-benzylidène-(phényl-1 éthylamine)-(S) et de 1,38 g de chlorure de benzyloxy-2 acétyle. On obtient ainsi 1,25 g d'un mélange en proportion molaire : 60/40 de deux diastéréoisomères de benzyloxy-3 phényl-4 [phényl-1-(S)]éthyl-1 azétidinone-2 sous forme d'une huile jaune.

### EXEMPLE 2

A une solution de 0,53 g d'amino-3 hydroxy-2 phényl-3 propionate-(2R,3S) de méthyle dans 8 cm3 de dichlorométhane, maintenue sous atmosphère d'argon, on ajoute 0,25 g d'hydrogénocarbonate de sodium puis goutte à goutte, à une température voisine de 20°C, une solution de 0,73 g de dicarbonate de di.tert-butyle dans 2 cm3 de dichlorométhane. La solution obtenue est agitée pendant 72 heures à une température voisine de 20°C puis additionnée de 20 cm3 d'eau distillée. La phase aqueuse est séparée par décantation puis réextraite par 2 fois 10 cm3 de dichlorométhane. Les phases organiques sont réunies, séchées sur sulfate de magnésium, filtrées puis concentrées à sec sous pression réduite (2,7 kPa) à 40°C.

On obtient ainsi après cristallisation dans l'oxyde de diisopropyle 0,45 g de tert-butoxycarbonylamino-3 hydroxy-2 phényl-3 propionate-(2R,3S) de méthyle sous forme de cristaux blancs fondant à 135°C dont les caractéristiques physiques sont identiques à celles décrites dans le brevet européen EP 0 414 610 :
- pouvoir rotatoire : [α]²⁰_{D} = -2,6° (c = 1 ; méthanol)
   [α]²⁰_{D} = -7,4° (c = 1,03 ; chloroforme)
- spectre de RMN (200 MHz ; CDCl₃)
   δ (ppm) : 1,42 (s, 9H : -NHCOOC(CH₃)₃) ; 3,16 (d, 1H, J = 5 Hz : -OH ; 3,87 (s, 3H : -COOCH₃); 4,48 (m, 1H : -CHOH) ; 5,22 (d large, 1H, J = 10,5 Hz : -CHNHCOOC(CH₃)₃) ; 5,39 (d, 1H, J = 10,5 Hz : -NHCOOC(CH₃)₃) ; 7,20 à 7,45. (m, 5H : -C₆H₅).

Le produit ainsi obtenu peut être transformé en taxotère dans les conditions décrites dans le brevet européen EP 0 336 841.

### EXEMPLE 3

A 1 g d'une dispersion à 10 % de palladium dans du charbon activé en poudre on ajoute une solution de 5,05 g d'hydroxy-2 [phényl-1-(S)]éthylamino-3 (fluoro-4 phényl)-3 propionate-(2R,3S) de méthyle dans un mélange de 95 cm3 de méthanol et de 32 cm3 d'acide acétique. Le mélange réactionnel est chauffé à une température de 65°C pendant 5 heures, sous agitation et sous une pression de 2300 kPa (23 bars) d'hydrogène, dans un autoclave de 1000 cm3 en acier inoxydable. Le mélange réactionnel est ensuite refroidi à une température voisine de 20°C et filtré sur verre fritté garni de célite. Le verre fritté est lavé par 3 fois 30 cm3 de méthanol et les filtrats sont réunis puis concentrés à sec sous pression réduite (2,7 kPa) à une température voisine de 40°C.

Le résidu est additionné de 50 cm3 d'eau distillée et la solution obtenue est alcalinisée à un pH voisin de 7 par addition d'une solution aqueuse 7,5N d'hydroxyde de sodium puis extraite par 3 fois 80 cm3 de dichlorométhane. Les phases organiques sont réunies, séchées sur sulfate de magnésium, filtrées puis concentrées à sec sous pression réduite (2,7 kPa) à 40°C. On obtient ainsi 2,45 g d'amino-3 hydroxy-2 (fluoro-4 phényl)-3 propionate-(2R,3S) de méthyle sous forme de cristaux crème fondants à 105°C dont les caractéristiques physiques sont les suivantes :
- spectre de R.M.N.: (300 MHz ; CDCl₃ + ε CD₃COOD ; δ en ppm). 3,56 (s, 3H : -COOCH₃); 4,61 et 4,69 (2 mt, 1H chacun : -CHOH et -CHNH₂); 7,06 [t, J = 8,5 Hz, 2H : -C₆H₄F(-H 3 et -H 5)]; 7,46 [dd, J = 8,5 et 6,5 Hz, 2H : -C₆H₄F(-H 2 et -H 6)].

L'hydroxy-2 [phényl-1-(S)] éthylamino-3 (fluoro-4 phényl)-3 propionate-(2R,3S) de méthyle peut être préparé de la manière suivante :

Une solution de 5,45 g d'hydroxy-3 (fluoro-4 phényl)-4 [phényl-1-(S)]éthyl-1 azétidinone-2-(3R,4S) dans un mélange de 175 cm3 de méthanol et de 35 cm3 d'une solution aqueuse 6N d'acide chlorhydrique est chauffée au reflux (65°C) pendant 18 heures, puis refroidie à une température voisine de 20°C et concentrée à sec sous pression réduite (2,7 kPa) à une température voisine de 40°C. Le résidu est additionné de 150 cm3 d'eau distillée et alcalinisé jusqu'à un pH voisin de 7 par addition d'une solution aqueuse 7,5N d'hydroxyde de sodium puis extrait par 3 fois 150 cm3 de dichlorométhane. Les phases organiques sont réunies, séchées sur sulfate de magnésium, filtrées puis concentrées à sec sous pression réduite (2,7 kPa) à 40°C. On obtient ainsi 5,08 g d'hydroxy-2 [(phényl-1(S)] éthylamino-3 (fluoro-4 phényl)-3 propionate-(2R,3S) de méthyle sous forme d'une huile jaune pâle dont les caractéristiques physiques sont les suivantes :
- spectre de R.M.N.: (300 MHz ; CDCl₃ ; δ en ppm). 1,26 (d, J = 7 Hz, 3H : -CHCH₃); 3,60 (q, J = 7 Hz, 1H : -CHCH₃); 3,79 (s, 3H : -COOCH₃) ; 4,12 et 4,19 (2d, J = 3 Hz, 1H chacun : -CHOH et -CHNH-); 7,00 [t, J = 8,5 Hz, 2H : -C₆H₄F(-H3 et -H 5)]; 7,10 à 7,40 [mt, 7H : -C₆H₅ et -C₆H₄F(-H 2 et -H6)].

L'hydroxy-3 (fluoro-4 phényl)-4 [phényl-1-(S)] éthyl-1 azétidinone-2-(3R,4S) peut être préparé de la manière suivante :

A un mélange de 470 cm3 d'une solution aqueuse 1N d'hydroxyde de potassium et de 250 cm3 de tétrahydrofuranne on ajoute en 75 minutes, sous agitation et à une température voisine de 0°C, une solution de 12,4 g d'un mélange en proportion molaire: 75/25 des deux diastéréoisomères d'acétoxy-3 (fluoro-4 phényl)-4 [phényl-1-(S)]éthyl-1 azétidinone-2 forme A et forme B dans 300 cm3 de tétrahydrofuranne. L'addition terminée le milieu réactionnel est agité à une température voisine de 0°C pendant 2,5 heures puis additionné de 250 cm3 d'une solution aqueuse saturée d'hydrogénocarbonate de sodium. La phase aqueuse est séparée par décantation et réextraite par 3 fois 250 cm3 d'acétate d'éthyle. Les phases organiques sont réunies, séchées sur sulfate de magnésium, filtrées puis concentrées à sec sous pression réduite (2,7 kPa) à 40°C.

On obtient ainsi 10,1 g de cristaux blancs que l'on recristallise dans 55 cm3 d'un mélange d'acétate d'éthyle et d'hexane (80-20 en volumes) pour donner 5,45 g d'hydroxy-3 (fluoro-4 phényl)-4 [phényl-1-(S)]éthyl-1 azétidinone-2-(3R,4S) sous forme de cristaux blancs fondants à 155°C et dont les caractéristiques physiques sont les suivantes :
- spectre de R.M.N.: (300 MHz ; CDCl₃ ; δ en ppm).
   1,29 (d, J = 7,5 Hz, 3H : -CHCH₃); 3,59 (s large, 1H : -OH); 4,40 (d, J = 3,5 Hz, 1H : -CHC₆H₄F); 4,52 (d large, J = 3,5 Hz, 1H : -CHOH); 4,90 (q, J = 7,5 Hz, 1H : -CHCH₃); 6,96 [t, J = 8,5 Hz, 2H : -C₆H₄F(-H 3 et -H 5)1; 7,00 à 7,30 [mt, 7H : -C₆H₅ et -C₆H₄F(-H 2 et -H6)].

Le mélange des formes A et B de l'acétoxy-3 (fluoro-4 phényl)-4 [phényl-1-(S)]éthyl-1 azétidinone-2 peut être préparé de la manière suivante :

A une solution de 16,8 g de N-(fluoro-4)benzylidène-(phényl-1 éthylamine)-(S) dans 220 cm3 de chloroforme on ajoute, sous agitation et à une température voisine de 20°C, 20,8 cm3 de triéthylamine puis on refroidit le mélange réactionnel jusqu'à une température voisine de -20°C et additionne goutte à goutte, en 1 heure et en se maintenant à cette température, une solution de 8,2 cm3 de chlorure d'acétoxy-2 acétyle dans 80 cm3 de chloroforme. La solution obtenue est agitée pendant 16 heures à une température voisine de 20°C puis additionnée de 200 cm3 d'une solution aqueuse 2,7N d'acide chlorhydrique. La phase organique est séparée par décantation, lavée par 2 fois 200 cm3 d'eau distillée puis par 200 cm3 d'une solution aqueuse saturée d'hydrogénocarbonate de sodium, séchée sur sulfate de magnésium, filtrée puis concentrée à sec sous pression réduite (2,7 kPa) à 40°C. On obtient ainsi 19,7 g d'une huile brune que l'on purifie par chromatographie sur 1100 g de silice (0,04-0,063 mm) contenus dans une colonne de 8,5 cm de diamètre [éluant: cyclohexane-acétate d'éthyle (70-30 en volumes)] en recueillant des fractions de 60 cm3. Les fractions ne contenant que le produit cherché sont réunies et concentrées à sec sous pression réduite (0,27 kPa) à 40°C. On obtient ainsi 13,7 g d'un mélange en proportion molaire 75/25 de deux diastéréoisomères d'acétoxy-3 (fluoro-4 phényl)-4 [phényl-1-(S)]éthyl-1 azétidinone-2 sous forme d'une huile jaune.

La N-(fluoro-4)benzylidène-(phényl-1 éthylamine)-(S) peut être préparée de la manière suivante :

A une solution de 12,4 g de fluoro-4 benzaldéhyde dans 80 cm3 de dichlorométhane on ajoute, sous agitation et à une température voisine de 20°C, 13 cm3 de phényl-1 éthylamine-(S) et 6 g de tamis moléculaire 4 Å. Le mélange réactionnel est agité pendant 16 heures à une température voisine de 20°C puis filtré sur verre fritté garni de célite. Le verre fritté est lavé par 3 fois 20 cm3 de dichlorométhane et les filtrats sont réunis puis concentrés à sec sous pression réduite (2,7 kPa) à une température voisine de 40°C. On obtient ainsi 18,3 g de N-(fluoro-4)benzylidène-(phényl-1 éthylamine)-(S) sous forme d'une huile opalescente.

### EXEMPLE 4

A une solution de 2,4 g d'amino-3 hydroxy-2 (fluoro-4 phényl)-3 propionate-(2R,3S) de méthyle dans 60 cm3 de dichlorométhane, maintenue sous atmosphère d'argon, on ajoute 0,95 g de carbonate de sodium puis goutte à goutte, à une température voisine de 20°C, une solution de 2,46 g de dicarbonate de di.tert-butyle dans 20 cm3 de dichlorométhane. La solution obtenue est agitée pendant 16 heures à une température voisine de 20°C puis additionnée de 100 cm3 d'eau distillée. La phase aqueuse est séparée par décantation puis réextraite par 2 fois 50 cm3 de dichlorométhane. Les phases organiques sont réunies, séchées sur sulfate de magnésium, filtrées puis concentrées à sec sous pression réduite (2,7 kPa) à 40°C.

On obtient ainsi après recristallisation dans l'oxyde de diisopropyle 2,35 g de tert-butoxycarbonylamino-3 hydroxy-2 (fluoro-4 phényl)-3 propionate-(2R,3S) de méthyle sous forme de cristaux blancs fondants à 125°C qui est transformé en tert-butoxycarbonylamino-3 hydroxy-2 (fluoro-4 phényl)-3 propionate-(2R,3S) d'acétoxy-4 benzoyloxy-2α époxy-5β,20 trihydroxy-1β,7β,10β oxo-9 taxène-11 yle-13α en opérant dans les conditions décrites dans le brevet européen EP 0 336 841.

### EXEMPLE 5

En opérant dans les conditions décrites dans l'exemple 1, on prépare l'amino-3 hydroxy-2 (trifluorométhyl-4 phényl)-3 propionate-(2R,3S) de méthyle sous forme de cristaux crème fondant à 134°C en passant par les intermédiaires suivants :
- l'hydroxy-2 [phényl-1-(S)]éthylamino-3 (trifluorométhyl-4 phényl)-3 propionate-(2R,3S) de méthyle sous forme d'une huile jaune,
- l'hydroxy-3 (trifluorométhyl-4 phényl)-4 [phényl-1-(S)]éthyl-1 azétidinone-2-(3R,4S) sous forme de cristaux blancs fondants à 165°C,
- le mélange des formes A et B de l'acétoxy-3 (trifluorométhyl-4 phényl)-4 [phényl-1-(S)]éthyl-1 azétidinone-2 sous forme d'une pâte blanche,
- la N-(trifluorométhyl-4)benzylidène-(phényl-1 éthylamine)-(S) sous forme de cristaux blancs fondants en dessous de 50°C

### EXEMPLE 6

En opérant comme dans l'exemple 2, mais à partir de 2,73 g d'amino-3 hydroxy-2 (trifluorométhyl-4 phényl)-3 propionate-(2R,3S) de méthyle, on obtient 2,43 g de tert-butoxycarbonylamino-3 hydroxy-2 (trifluorométhyl-4 phényl)-3 propionate-(2R,3S) de méthyle sous forme de cristaux blancs fondant à 120°C dont les caractéristiques physiques sont les suivantes :
- spectre de R.M.N.: (300 MHz ; DMSO d₆ ; δ en ppm).
   1,40 [s, 9H : -NHCOOC(CH₃)₃]; 3,62 (s, 3H : -COOCH₃); 4,40 (mt, 1H : -CHOH); 5,08 [(dd, J = 11 et 4,5 Hz, 1H : -CHNHCOOC(CH₃)₃]; 5,65 (d, J = 6,5 Hz, 1H : -OH); 7,40 [d, J = 11 Hz, 1H : -NHCOOC(CH₃)₃]; 7,59 et 7,72 (2d, J = 8,5 Hz, 2H chacun : -C₆H₄CF₃(-H3, -H5 et -H2, -H6)].

Le produit ainsi obtenu est transformé en tert-butoxycarbonylamino-3 hydroxy-2 (trifluorométhyl-4 phényl)-3 propionate-(2R,3S) d'acétoxy-4 benzoyloxy-2a époxy-5β,20 trihydroxy-1β,7β,10β oxo-9 taxène-11 yle-13α en opérant dans les conditions décrites dans le brevet européen EP 0 336 841.

### EXEMPLE 7

En opérant dans les conditions décrites dans l'exemple 1, on prépare l'amino-3 hydroxy-2 (diméthylamino-4 phényl)-3 propionate-(2R,3S) de méthyle sous forme de cristaux crème fondant à 119°C en passant par les intermédiaires suivants :
- l'hydroxy-2 [phényl-1-(S)]éthylamino-3 (diméthylamino-4 phényl)-3 propionate-(2R,3S) de méthyle sous forme de cristaux blancs fondant à 122°C,
- l'hydroxy-3 (diméthylamino-4 phényl)-4 [phényl-1-(S)]éthyl-1 azétidinone-2-(3R,4S) sous forme de cristaux blancs fondants à 220°C,
- le mélange des formes A et B de l'acétoxy-3 (diméthylamino-4 phényl)-4 [phényl-1-(S)]éthyl-1 azétidinone-2 sous forme de cristaux blancs fondant à 136°C,
- la N-(diméthylamino-4)benzylidène-(phényl-1 éthylamine)-(S) sous forme de cristaux blancs fondants en dessous de 50°C.

### EXEMPLE 8

En opérant comme dans l'exemple 2, mais à partir de 0,8 g d'amino-3 hydroxy-2 (diméthylamino-4 phényl)-3 propionate-(2R,3S) de méthyle, on obtient 0,82 g de tert-butoxycarbonylamino-3 hydroxy-2 (diméthylamino-4 phényl)-3 propionate-(2R,3S) de méthyle sous forme de cristaux blancs fondants à 120°C dont les caractéristiques physiques sont les suivantes :
Spectre de R.M.N.: (200 MHz; DMSO d6 ; δ en ppm).
1,39 [s, 9H : -NHCOOC(CH3)3]; 2,90 [s, 6H : -N(CH3)2]; 3,59 (s, 3H : -COOCH3); 4,21 (dd, J = 7,5 et 4,5 Hz, 1H : -CHOH); 4,81 [dd, J = 9,5 et 4,5 Hz, 1H : -CHNHCOOC(CH3)3]; 5,47 (d, J = 7,5 Hz, 1H : -OH); 7,02 [d, J = 9,5 Hz, 1H : -NHCOOC(CH3)3]; 6,66 [d, J = 8,5 Hz, 2H : -C6H4N(CH3)2(-H 3 et -H 5)]; 7,12 [2d, J = 8,5 Hz, 2H : -C6H4N(CH3)2(-H 2 et -H 6)].

Le produit ainsi obtenu est transformé en tert-butoxycarbonylamino-3 hydroxy-2 (diméthylamino-4 phényl)-3 propionate-(2R,3S) d'acétoxy-4 benzoyloxy-2α époxy-5β,20 trihydroxy-1β,7β,10β oxo-9 taxène-11 yle-13α en opérant dans les conditions décrites dans le brevet européen EP 0 336 841.

### EXEMPLE 9

A 2,1 g d'une dispersion à 20 % de dihydroxyde de palladium dans du charbon activé en poudre on ajoute une solution de 5,5 g de (carbamoyl-4 phényl)-3 hydroxy-2 [phényl-1-(S)]éthylamino-3 propionate-(2R,3S) de méthyle dans un mélange de 100 cm3 de méthanol et de 3 cm3 d'acide acétique. Le mélange réactionnel est maintenu sous agitation pendant 60 heures à une température voisine de 20°C et sous une pression de 120 kPa (1,2 bars) d'hydrogène. Le mélange réactionnel est ensuite filtré sur verre fritté garni de célite. Le verre fritté est lavé par 3 fois 15 cm3 de méthanol et les filtrats sont réunis puis concentrés à sec sous pression réduite (2,7 kPa) à une température voisine de 40°C. Les cristaux recueillis sont lavés sur verre fritté par 20 cm3 d'oxyde de diéthyle.

On obtient ainsi 4,6 g d'acétate de l'amino-3 (carbamoyl-4 phényl)-3 hydroxy-2 propionate-(2R,3S) de méthyle sous forme de cristaux blancs fondant à 206°C et dont les caractéristiques sont les suivantes :
- spectre de R.M.N.: (300 MHz ; DMSO d₆ ; δ en ppm).
   1,90 (s, 3H : CH₃COO-); 3,60 (s, 3H : -COOCH₃); 4,13 (ab limite, 2H : -CHOH et -CHNH⁺₃); 7,30 et 7,95 (2s, 1H chacun : -CONH₂); 7,40 [(d, J = 8,5 Hz, 2H : -C₆H₄CONH₂(-H2 et -H6)]; 7,80 (d, J = 8,5 Hz, 2H : -C₆H₄CONH₂(-H 3 et -H 5)].

Le (carbamoyl-4 phényl)-3 hydroxy-2 [phényl-1-(S)]éthylamino-3 propionate-(2R,3S) de méthyle peut être préparé de la manière suivante :

A une solution de 10 g de (cyano-4 phényl)-4 hydroxy-3 [phényl-1-(S)] éthyl-1 azétidinone-2-(3R,4S) dans 100 cm3 d'acide acétique on ajoute 10,9 g d'acétate mercurique. Le milieu réactionnel est chauffé au reflux pendant 5 heures, puis refroidi à une température voisine de 20°C et concentré à sec sous pression réduite (2,7 kPa) à une température voisine de 40°C. Le résidu est additionné de 150 cm3 de méthanol et concentré à sec sous pression réduite (2,7 kPa) à une température voisine de 40°C. Au solide résiduel on ajoute 300 cm3 de méthanol et injecte, sous agitation, dans le milieu réactionnel un courant gazeux d'acide chlorhydrique anhydre à une température voisine de 40°C pendant 1,5 heure. Le milieu réactionnel est concentré à sec sous pression réduite (2,7 kPa) à 40°C puis versé dans un mélange de 300 cm3 d'acétate d'éthyle, 300 cm3 d'eau distillée et 100 cm3 d'une solution aqueuse saturée d'hydrogénocarbonate de sodium. La phase aqueuse est séparée par décantation et réextraite par 2 fois 250 cm3 d'acétate d'éthyle. Les phases organiques sont réunies, lavées par 2 fois 150 cm3 d'une solution aqueuse à 3 % de sulfure de sodium puis par 2 fois 100 cm3 d'eau distillée, séchées sur sulfate de magnésium, filtrées puis concentrées à sec sous pression réduite (2,7 kPa) à 40°C. Les cristaux recueillis sont lavés sur verre fritté par 20 cm3 d'oxyde de diéthyle. On obtient ainsi 5,5 g de (carbamoyl-4 phényl)-3 hydroxy-2 [(phényl-1(S)]éthylamino-3 propionate- (2R,3S) de méthyle sous forme de cristaux blancs fondant à 130°C dont les caractéristiques sont les suivantes :
- spectre de R.M.N.: (300 MHz ; CDCl₃ ; δ en ppm)
   1,30 (d, J = 7 Hz, 3H : -CHCH₃) ; 3,65 (q, J = 7 Hz, 1H : -CHCH₃) ; 3,85 (s, 3H : -COOCH₃) ; 4,25 et 4,35 (2d, J = 3,5 Hz, 1H chacun : -CHOH- et -CHNH-) ; 5,97 et 6,17 (2mf, 1H chacun : -CONH₂) ; 7,20 à 7,40 (mt, 5H : -C₆H₅) ; 7,41 [d, J = 8,5 Hz, 2H : -C₆H₄CONH₂(H 2 et H 6)] ; 7,81 [d, J = 8,5 Hz, 2H : -C₆H₄CONH₂(H 3 et H 5)].

Le (cyano-4 phényl)-4 hydroxy-3 [phényl-1-(S)]éthyl-1 azétidinone-2-(3R,4S) peut être préparé de la manière suivante :

A une solution de 55,3 g d'un mélange en proportion molaire 65/35 des deux diastéréoisomères d'acétoxy-3 (cyano-4 phényl)-4 [phényl-1-(S)]éthyl-1 azétidinone-2 forme A et forme B dans 550 cm3 de méthanol on injecte, sous agitation, un courant gazeux d'ammoniac anhydre à une température voisine de 0°C pendant 3 heures. Le milieu réactionnel est concentré à sec sous pression réduite (2,7 kPa) à 40°C.

On obtient 41 g de cristaux blancs que l'on recristallise dans un mélange de 280 cm3 d'acétate d'éthyle et de 70 cm3 d'oxyde de diéthyle. Les cristaux obtenus sont recristallisés une seconde fois dans 160 cm3 d'acétate d'éthyle puis une troisième fois dans 100 cm3 d'acétonitrile. On obtient ainsi 10 g de (cyano-4 phényl)-4 hydroxy-3 [phényl-1-(S)]éthyl-1 azétidinone-2-(3R,4S) sous forme de cristaux blancs fondants à 139°C et dont les caractéristiques sont les suivantes :
- spectre de R.M.N.: (300 MHz ; CDCl₃ ; δ en ppm).
   1,39 (d, J = 7,5 Hz, 3H : -CHCH₃) ; 3,89 (d, J = 6,5 Hz, 1H : -OH) ; 4,54 ( d, J = 4 Hz, 1H : -CHC₆H₅) ; 4,96 (dd, J = 6,5 et 4 Hz, 1H : -CHOH) ; 4,96 (q, J = 7,5 Hz, 1H : -CHCH₃) ; 7,10 à 7,40 (mt, 5H : -C₆H₅) ; 7,43 [d, J = 8,5 Hz, 2H : -C₆H₄CN(-H 2 et -H6)] ; 7,66 [d, J = 8,5 Hz, 2H : -C₆H ₄CN(-H 3 et -H 5)].

Le mélange des formes A et B de l'acétoxy-3 (cyano-4 phényl)-4 [phényl-1-(S)]éthyl-1 azétidinone-2 peut être préparé de la manière suivante :

A une solution de 56,1 g de N-(cyano-4)benzylidène-(phényl-1 éthylamine)-(S) dans 600 cm3 de chloroforme on ajoute, sous agitation et à une température voisine de 0°C, 47,6 cm3 de triéthylamine puis, goutte à goutte, en 3 heures et en se maintenant à cette température, une solution de 18,6 cm3 de chlorure d'acétoxy-2 acétyle dans 500 cm3 de chloroforme. La solution obtenue est agitée pendant 16 heures à une température voisine de 20°C puis additionnée de 250 cm3 d'eau distillée. La phase organique est séparée par décantation, lavée par 250 cm3 d'eau distillée, séchée sur sulfate de magnésium, filtrée puis concentrée à sec sous pression réduite (2,7 kPa) à 40°C. On obtient ainsi 76 g d'une huile brune que l'on purifie par chromatographie sur 3500 g de silice (0,04-0,063 mm) contenus dans une colonne de 15 cm de diamètre (éluant: dichlorométhane). Les fractions ne contenant que le produit cherché sont réunies et concentrées à sec sous pression réduite (0,27 kPa) à 40 C. On obtient ainsi 55,3 g d'un mélange en proportion molaire 65/35 de deux diastéréoisomères d'acétoxy-3 (cyano-4 phényl)-4 [phényl-1-(S)]éthyl-1 azétidinone-2 sous forme d'une huile opalescente.

La N-(cyano-4)benzylidène-(phényl-1 éthylamine)-(S) peut être préparée de la manière suivante :

A une solution de 25 g de cyano-4 benzaldéhyde dans 200 cm3 de dichlorométhane on ajoute, sous agitation et à une température voisine de 20°C, 24,3 cm3 de phényl-1 éthylamine-(S) et 12 g de tamis moléculaire 4 Å. Le mélange réactionnel est agité pendant 16 heures à une température voisine de 20°C puis filtré sur verre fritté garni de célite. Le verre fritté est lavé par 3 fois 50 cm3 de dichlorométhane et les filtrats sont réunis puis concentrés à sec sous pression réduite (2,7 kPa) à une température voisine de 40°C. On obtient ainsi 41,4 g de N-(cyano-4)benzylidène-(phényl-1 éthylamine)-(S) sous forme d'une huile incolore.

### EXEMPLE 10

A une solution de 2,2 g d'amino-3 (carbamoyl-4 phényl)-3 hydroxy-2 propionate-(2R,3S) de méthyle dans 50 cm3 de tétrahydrofuranne, maintenue sous atmosphère d'argon, on ajoute à une température voisine de 20°C, 1,24 g d'hydrogénocarbonate de sodium puis 1,62 g de dicarbonate de di.tert-butyle. Le milieu réactionnel est agité pendant 48 heures à une température voisine de 20°C puis concentré à sec sous pression réduite (2,7 kPa) à 40°C et additionné de 20 cm3 d'eau distillée. Le solide formé est séparé par filtration, lavé par 10 cm3 d'eau distillée puis 10 cm3 d'oxyde de diisopropyle et séché à l'air ambiant.

On obtient ainsi 2,1 g de tert-butoxycarbonylamino-3 (carbamoyl-4 phényl)-3 hydroxy-2 propionate-(2R,3S) de méthyle sous forme de cristaux blancs fondants à 232°C dont les caractéristiques physiques sont les suivantes :
- spectre de R.M.N.: (300 MHz ; DMSO d₆ ; δ en ppm).
   1,41 [s, 9H : -NHCOOC(CH₃)₃] ; 3,62 (s, 3H : -COOCH₃) ; 4,38 (d, J = 4,5 Hz, 1H: -CHOH); 5,02 [dd, J = 10 et 4,5 Hz, 1H : -CHNHCOOC(CH₃)₃] ; 5,65 (mf étalé, 1H : -OH) ; 7,32 [d, J = 10 Hz, 1H : -NHCOOC(CH₃)₃] ; 7,39 et 8,00 (2s, 1H chacun : -CONH₂) ; 7,41 [d, J = 8,5 Hz, 2H : -C₆H ₄CONH₂(-H 2 et -H6)] ; 7,84 [d, J = 8,5 Hz, 2H : -C₆H₄CONH₂(-H 3 et -H 5)].

Le produit ainsi obtenu est transformé en tert-butoxycarbonylamino-3 hydroxy-2 (carbamoyl-4 phényl)-3 propionate-(2R,3S) d'acétoxy-4 benzoyloxy-2α époxy-5β,20 trihydroxy-1β,7β,10β oxo-9 taxène-11 yle-13α en opérant dans les conditions décrites dans le brevet européen EP 0 336 841.

### EXEMPLE 11

A un mélange de 1,8 g de tert-butoxycarbonylamino-3 (carbamoyl-4 phényl)-3 hydroxy-2 propionate-(2R,3S) de méthyle et de 15 cm3 de pyridine anhydre, maintenue sous atmosphère d'argon, on ajoute goutte à goutte, à une température voisine de 20°C, 1,97 cm3 de triéthylchlorosilane. L'addition terminée le milieu réctionnel est agité pendant 3 heures à une température voisine de 20°C puis versé dans un mélange de 200 cm3 d'eau distillée et de 50 cm3 de dichlorométhane. La phase aqueuse est séparée par décantation puis réextraite par 3 fois 50 cm3 de dichlorométhane. Les phases organiques sont réunies, séchées sur sulfate de magnésium, filtrées puis concentrées à sec sous pression réduite (2,7 kPa) à 40°C et les cristaux recueillis sont lavés sur verre fritté par 20 cm3 d'oxyde de diisopropyle.

On obtient ainsi 1,92 g de tert-butoxycarbonylamino-3 (carbamoyl-4 phényl)-3 triéthylsilyloxy-2 propionate-(2R,3S) de méthyle sous forme de cristaux blancs fondants à 165°C dont les caractéristiques physiques sont les suivantes :
- spectre de R.M.N.: (300 MHz ; DMSO d₆ ; δ en ppm).
   0,43 (mt, 6H : -OSi(CH₂CH₃)₃ ; 0,80 (t, J = 7,5 Hz, 9H : -OSi(CH₂CH₃)₃) ; 1,40 [s, 9H -NHCOOC(CH₃)₃] ; 3,56 (s, 3H : -COOCH₃) ; 4,42 (d, J = 4,5 Hz, 1H ; -CHOH) ; 5,04 [dd, J = 9,5 et 4 ,5 Hz, 1H : -CHNHCOOC(CH₃)₃] ; 7,33 [d, J = 9,5 Hz, 1H : -NHCOOC(CH₃)₃] ; 7,37 et 7,98 (2s, 1H chacun : -CONH₂) ; 7,46 [d, J = 8,5 Hz, 2H : -C₆H₄CONH₂(-H 2 et -H 6)] ; 7,84 (d, J = 8,5 Hz, 2H : -C₆H₄CONH₂(-H 3 et -H 5)].

A une solution de 1,18 g de tert-butoxycarbonylamino-3 (carbamoyl-4 phényl)-3 triéthylsilyloxy-2 propionate-(2R,3S) de méthyle dans 20 cm3 de pyridine anhydre, maintenue sous atmosphère d'argon, on ajoute goutte à goutte, à une température voisine de 0°C, 0,24 cm3 d'oxychlorure de phosphore. L'addition terminée le milieu réctionnel est agité pendant 3 heures à une température voisine de 0°C puis versé dans un mélange de 100 cm3 d'eau distillée et de 100 cm3 d'une solution aqueuse saturée d'hydrogénocarbonate de sodium. Après 5 minutes d'agitation à une température voisine de 20°C, on ajoute 100 cm3 de dichlorométhane puis sépare la phase aqueuse et la réextrait par 2 fois 80 cm3 de dichlorométhane. Les phases organiques sont réunies, séchées sur sulfate de magnésium, filtrées puis concentrées à sec sous pression réduite (2,7 kPa) à 40°C.

Le solide résiduel est dissous dans 30 cm3 de méthanol et additionné de 3 cm3 d'une solution aqueuse 1N d'acide chlorhydrique. La solution obtenue est agitée pendant 45 minutes à une température voisine de 20°C puis concentrée à sec sous pression réduite (2,7 kPa) à 40°C. Le solide résiduel est additionné de 50 cm3 d'une solution aqueuse saturée d'hydrogénocarbonate de sodium puis est extrait par 3 fois 30 cm3 d'acétate d'éthyle. Les phases organiques sont réunies, séchées sur sulfate de magnésium, filtrées puis concentrées à sec sous pression réduite (2,7 kPa) à 40°C. Les cristaux recueillis sont lavés sur verre fritté par 20 cm3 d'oxyde de diisopropyle.

On obtient ainsi 0,75 g de tert-butoxycarbonylamino-3 (cyano-4 phényl)-3 hydroxy-2 propionate-(2R,3S) de méthyle sous forme de cristaux blancs fondant à 250°C dont les caractéristiques physiques sont les suivantes :
- spectre de R.M.N.: (200 MHz ; DMSO d₆ ; δ en ppm).
   1,39 [s, 9H : -NHCOOC(CH₃)₃] ; 3,62 (s, 3H : -COOCH₃); 4,39 (dd, J = 8 et 4,5 Hz, 1H : -CHOH); 5,05 [dd, J = 9,5 et 4,5 Hz, 1H : -CHNHCOOC(CH₃)₃] ; 5,65 (d, J = 8 Hz, 1H : -OH) ; 7,40 [d, J = 9,5 Hz, 1H : -NHCOOC(CH₃)₃] ; 7,53 [d, J = 8,5 Hz, 2H : -C₆H ₄CN(-H 2 et -H6)] ; 7,82 (d, J = 8,5 Hz, 2H : -C₆H ₄CN(-H 3 et -H5)].

Le produit ainsi obtenu est transformé en tert-butoxycarbonylamino-3 hydroxy-2 (cyano-4 phényl)-3 propionate-(2R,3S) d'acétoxy-4 benzoyloxy-2α époxy-5β,20 trihydroxy-1β,7β,10β oxo-9 taxène-11 yle-13α en opérant dans les conditions décrites dans le brevet européen EP 0 336 841.

## Revendications

1. Procédé de préparation de la β-phénylisosérine et de ses analogues de formule générale : 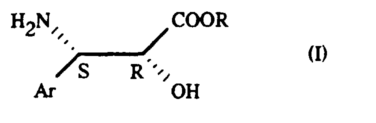 dans laquelle Ar représente un radical aryle et R représente un atome d'hydrogène ou un radical alcoyle contenant 1 à 4 atomes de carbone, éventuellement substitué par un radical phényle, ou un radical phényle, caractérisé en ce que l'on effectue l'hydrogénolyse d'un produit de formule générale : 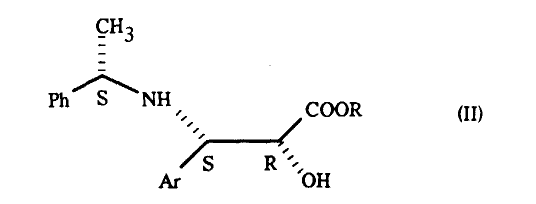 dans laquelle Ar et R sont définis comme précédemment et Ph représente un radical phényle éventuellement substitué par un ou plusieurs atomes ou radicaux choisis parmi les atomes d'halogène et les radicaux alcoxy contenant 1 à 4 atomes de carbone, alcoylthio contenant 1 à 4 atomes de carbone, amno, alcoylamino contenant 1 à 4 atomes de carbone, dialcoylamino dont chaque partie alcoyle contient 1 à 4 atomes de carbone ou nitro.

2. Procédé selon la revendication 1 caractérisé en ce que l'hydrogénolyse est effectuée au moyen d'hydrogène en présence d'un catalyseur.

3. Procédé selon la revendication 2 caractérisé en ce que le catalyseur est constitué par du palladium sur charbon ou du dihydroxyde de palladium sur charbon.

4. Procédé selon l'une des revendications 2 ou 3 caractérisé en ce que l'on opère sous une pression d'hydrogène comprise entre 1 et 50 bars.

5. Procédé selon l'une des revendications 2, 3 ou 4 caractérisé en ce que l'on opère dans un solvant organique.

6. Procédé selon la revendication 5 caractérisé en ce que le solvant organique est l'acide acétique éventuellement associé à un alcool aliphatique contenant 1 à 4 atomes de carbone.

7. Procédé selon la revendication 1 caractérisé en ce que le produit de formule générale (II) mis en oeuvre est obtenu par hydrolyse ou alcoolyse d'un produit de formule générale : 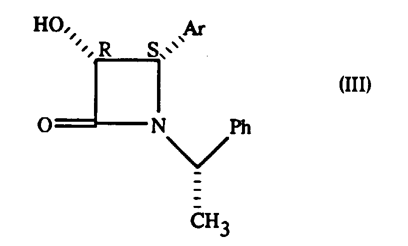 dans laquelle Ar et Ph sont définis comme dans la revendication 1, au moyen d'un alcool en milieu acide, le produit de formule générale (III) étant obtenu par saponification ou hydrogénolyse d'un produit de formule générale : 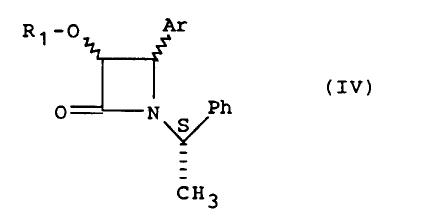 dans laquelle Ar et Ph sont définis comme dans la revendication 1 et R₁ représente un groupement protégeant la fonction alcool sous forme d'ester ou d'éther, au moyen d'une base minérale ou par l'hydrogène éventuellement généré in situ,en présence d'un catalyseur choisi parmi le palladium sur noir contenant 1 à 10% en poids de palladium, suivie de la séparation des diastéréoisomères obtenus, le produit de formule générale (IV) étant obtenu par cycloaddition d'une imine de formule générale: 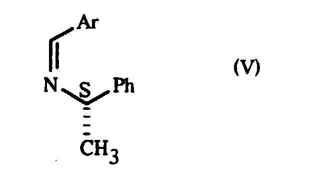 dans laquelle Ar et Ph sont définis comme précédemment, sur un halogénure d'acide de formule générale : 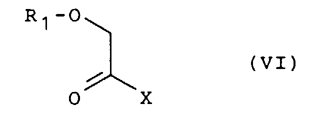 dans laquelle R₁ est défini comme précédemment et X représente un atome d'halogène choisi parmi le brome et le chlore, en présence d'une base organique choisie parmi les amines tertiaires aliphatiques et la pyridine.

8. Le produit de formule générale : 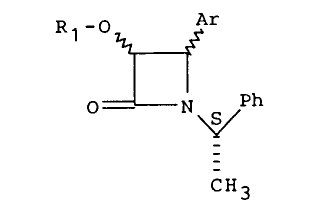 dans laquelle Ar représente un radical phényle ou α- ou β-naphtyle éventuellement substitué par un ou plusieurs atomes ou radicaux choisis parmi les atomes d'halogène (fluor, chlore, brome, iode) et les radicaux alcoyles, aryles, aralcoyles, alcoxy, alcoylthio, aryloxy, arylthio, hydroxy, hydroxyalcoyle, mercapto, formyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone et que les radicaux aryles sont des radicaux phényles ou α- ou β-naphtyles, Ph représente un radical phényle éventuellement substitué par un ou plusieurs atomes ou radicaux choisis parmi les atomes d'halogène et les radicaux alcoxy contenant 1 à 4 atomes de carbone, alcoylthio contenant 1 à 4 atomes de carbone, amno, alcoylamino contenant 1 à 4 atomes de carbone, dialcoylamino dont chaque partie alcoyle contient 1 à 4 atomes de carbone ou nitro et R₁ représente un radical alcoyle, phénylalcoyle ou phényle ou un radical R'₁-CO dans lequel R'₁ représente un radical alcoyle, phénylalcoyle ou phényle, étant entendu que, lorsque Ar représente un radical phényle, Ph est différent d'un radical phényle non substitué.

## Patentansprüche

1. Verfahren zur Herstellung von β-Phenylisoserin und seinen Analogen der allgemeinen Formel: 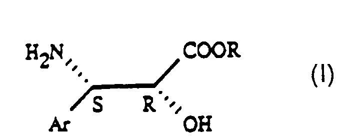 in der Ar einen Arylrest darstellt und R ein Wasserstoffatom oder einen gegebenenfalls mit einem Phenylrest substituierten Alkylrest mit 1 bis 4 Kohlenstoffatomen oder einen Phenylrest darstellt, dadurch gekennzeichnet, daß man die Hydrogenolyse eines Produkts der allgemeinen Formel: 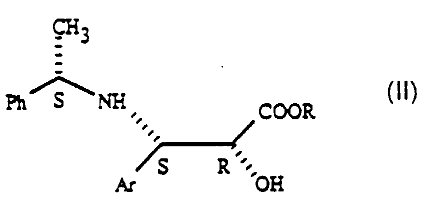 ausführt, in der Ar und R wie vorhergehend definiert sind und Ph einen Phenylrest darstellt, der gegebenenfalls mit einem oder mehreren Atomen oder Resten substituiert ist, die unter den Halogenatomen und den Alkoxyresten mit 1 bis 4 Kohlenstoffatomen, den Alkylthioresten mit 1 bis 4 Kohlenstoffatomen, dem Aminorest, den Alkylaminoresten mit 1 bis 4 Kohlenstoffatomen, den Dialkylaminoresten, von denen jeder Alkylteil 1 bis 4 Kohlenstoffatome enthält, oder dem Nitrorest ausgewählt sind.

2. Verfahren gemäß Anspruch 1, dadurch gekennzeichnet, daß die Hydrogenolyse mittels Wasserstoff in Gegenwart eines Katalysators ausgeführt wird.

3. Verfahren gemäß Anspruch 2, dadurch gekennzeichnet, daß der Katalysator aus Palladium auf Kohle oder Palladiumdihydroxid auf Kohle besteht.

4. Verfahren gemäß einem der Ansprüche 2 oder 3, dadurch gekennzeichnet, daß man unter einem Wasserstoffdruck zwischen 1 und 50 bar arbeitet.

5. Verfahren gemäß einem der Ansprüche 2, 3 oder 4, dadurch gekennzeichnet, daß man in einem organischen Lösungsmittel arbeitet.

6. Verfahren gemäß Anspruch 5, dadurch gekennzeichnet, daß das organische Lösungsmittel Essigsäure, gegebenenfalls in Verbindung mit einem aliphatischen Alkohol mit 1 bis 4 Kohlenstoffatomen, ist.

7. Verfahren gemäß Anspruch 1, dadurch gekennzeichnet, daß das eingesetzte Produkt der allgemeinen Formel (II) durch Hydrolyse oder Alkoholyse eines Produkts der allgemeinen Formel: 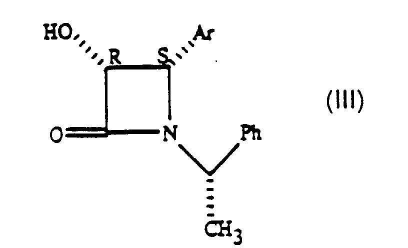 in der Ar und Ph wie in Anspruch 1 definiert sind, mittels eines Alkohols in saurem Medium erhalten wird, wobei das Produkt der allgemeinen Formel (III) durch Verseifung oder Hydrogenolyse eines Produkts der allgemeinen Formel: 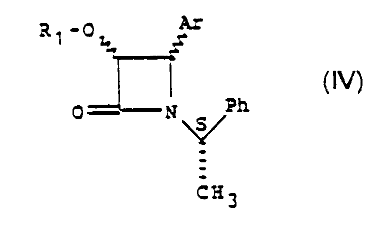 in der Ar und Ph wie in Anspruch 1 definiert sind und R₁ eine Schutzgruppe für die Alkoholfunktion in Form eines Esters oder Ethers darstellt, mittels einer Mineralbase oder durch gegebenenfalls in situ erzeugten Wasserstoff in Gegenwart eines unter Palladium auf Kohle mit 1 bis 10 Gew.-% Palladium ausgewählten Katalysators und der anschließenden Trennung der erhaltenen Diastereomere erhalten wird, wobei das Produkt der allgemeinen Formel (IV) durch Cycloaddition eines Imins der allgemeinen Formel: 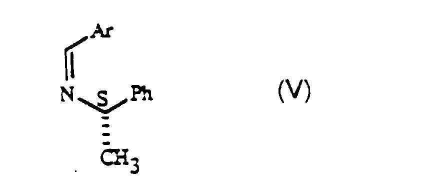 in der Ar und Ph wie vorhergehend definiert sind, an ein Säurehalogenid der allgemeinen Formel 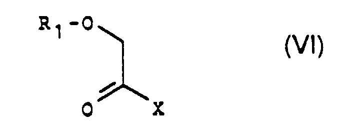 in der R₁ wie vorhergehend definiert ist und X ein unter Brom und Chlor ausgewähltes Halogenatom darstellt, in Gegenwart einer organischen Base, die unter den aliphatischen tertiären Aminen und Pyridin ausgewählt ist, erhalten wird.

8. Das Produkt der allgemeinen Formel: 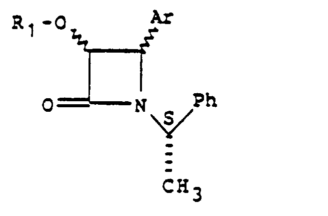 in der Ar einen Phenyl- oder α- oder β-Naphthylrest darstellt, der gegebenenfalls mit einem oder mehreren Atomen oder Resten substituiert ist, die unter den Halogenatomen (Fluor, Chlor, Brom, lod) und den Alkyl-, Aryl-, Aralkyl-, Alkoxy-, Alkylthio-, Aryloxy-, Arylthio-, Hydroxy-, Hydroxyalkyl-, Mercapto-, Formyl-, Acylamino-, Aroylamino-, Alkoxycarbonylamino-, Amino-, Alkylamino-, Dialkylamino-, Carboxy-, Alkoxycarbonyl-, Carbamoyl-, Dialkylcarbamoyl-, Cyano-, Nitro- und Trifluormethylresten ausgewählt sind, wobei es sich versteht, daß die Alkylreste und die Alkylteile der anderen Reste 1 bis 4 Kohlenstoffatome enthalten und daß die Arylreste Phenyl- oder α- oder β-Naphthylreste sind, Ph einen Phenylrest darstellt, der gegebenenfalls mit einem oder mehreren Atomen oder Resten substituiert ist, die unter den Halogenatomen und den Alkoxyresten mit 1 bis 4 Kohlenstoffatomen, den Alkylthioresten mit 1 bis 4 Kohlenstoffatomen, dem Aminorest, den Alkylaminoresten mit 1 bis 4 Kohlenstoffatomen, den Dialkylaminoresten, von denen jeder Alkylteil 1 bis 4 Kohlenstoffatome enthält, oder dem Nitrorest ausgewählt sind, und R₁ einen Alkyl-, Phenylalkyl- oder Phenylrest oder einen Rest R'₁-CO darstellt, in dem R'₁ einen Alkyl-, Phenylalkyl- oder Phenylrest darstellt, wobei es sich versteht, daß, wenn Ar einen Phenylrest darstellt, Ph von einem nicht substituierten Phenylrest verschieden ist.

## Claims

1. Process for the preparation of β-phenyl-isoserine and its analogues of general formula: 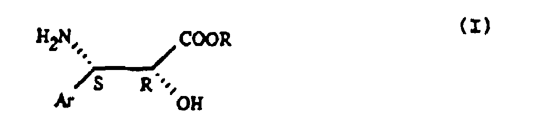 in which Ar represents an aryl radical and R represents a hydrogen atom or an alkyl radical containing 1 to 4 carbon atoms, which is optionally substituted by a phenyl radical, or a phenyl radical, characterised in that there is hydrogenolysed a product of general formula: 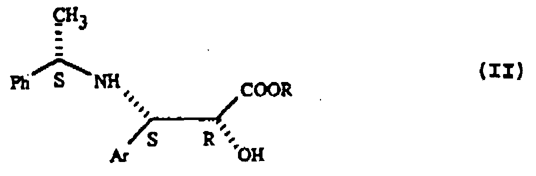 in which Ar and R are defined as above and Ph represents a phenyl radical which to optionally substituted by one or more atoms or radicals chosen from halogen and alkoxy radicals containing 1 to 4 carbon atoms, alkylthio radicals containing 1 to 4 carbon aroms, amino radicals, alkylamino radicals containing 1 to 4 carbon atoms, dialkylamino radicals in which each alkyl portion contains 1 to 4 carbon atoms or nitro radicals.

2. Process according to claim 1, characterised in that the hydrogenolysis is performed by means of hydrogen in the presence of a catalyst.

3. Process according to claim 2, characterised in that the catalyst consists of palladium on carbon or palladium dihydroxide on carbon.

4. Process according to one of claims 2 or 3, characterised in that the procedure is carried out at a hydrogen pressure of between 1 and 50 bars.

5. Process according to one of claims 2, 3 or 4, characterised in that the procedure is carried out in an organic solvent.

6. Process according to claim 5, characterised in that the organic solvent is acetic acid optionally combined with an aliphatic alcohol containing 1 to 4 carbon atoms.

7. Process according to claim 1, characterised in that the product of general formula (II) used is obtained by hydrolysis or alcoholysis of a product of general formula: 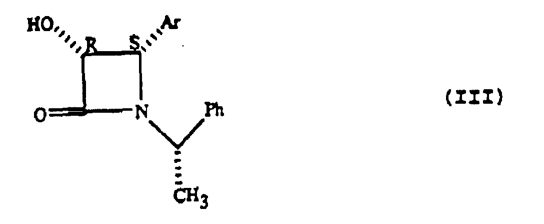 in which Ar and Ph are defined as in claim 1, by means of an alcohol in an acidic medium, the product of general formula (III) being obtained by saponification or hydrogenolysis of a product of general formula: 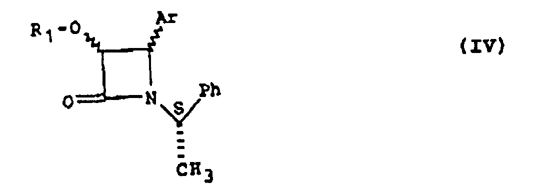 in which Ar and Ph are defined as in claim 1 and R₁ represents a group protecting the alcohol functional group in the form of an ester or an ether, by means of an inorganic base or by the hydrogen optionally generated in situ, in the presence of a catalyst chosen from palladium on black containing 1 to 10 % by weight of palladium, followed by separation of the diastereoisomers obtained, the product of general formula (IV) being obtained by cycloaddition of an imine of general formula: 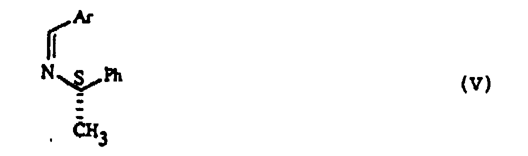 in which Ar and Ph are defined as above, onto an acid halide of general formula: 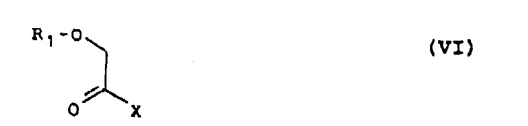 in which R₁ is defined as above and X represents a halogen atom chosen from bromine and chlorine, in the presence of an organic base chosen from aliphatic tertiary amines and pyridine.

8. The product of general formula: 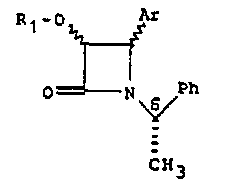 in which Ar represents a phenyl or an α- or β-naphthyl radical which is optionally substituted by one or more atoms or radicals chosen from halogen atoms (fluorine, chlorine, bromine, iodine) and alkyl, aryl, aralkyl, alkoxy, alkylthio, aryloxy, arylthio, hydroxy, hydroxyalkyl, mercapto, formyl, acylamino, aroylamino, alkoxycarbonylamino, amino, alkylamino, dialkylamino, carboxy, alkoxycarbonyl, carbamoyl, dialkylcarbamoyl, cyano, nitro and trifluoromethyl radicals, it being understood that the alkyl radicals and the alkyl portions of the other radicals contain 1 to 4 carbon atoms and that the aryl radicals are phenyl or α- or β-naphthyl radicals, Ph represents a phenyl radical which is optionally substituted by one or more atoms or radicals chosen from halogen and alkoxy radicals containing 1 to 4 carbon atoms, alkylthio radicals containing 1 to 4 carbon atoms, amino radicals, alkylamino radicals containing 1 to 4 carbon atoms, dialkylamino radicals in which each alkyl portion contains 1 to 4 carbon atoms or nitro radicals and R₁ represents an alkyl, phenylalkyl or phenyl radical or a R'₁-CO radical in which R'₁ represents an alkyl, phenylalkyl or phenyl radical, it being understood that, when Ar represents a phenyl radical, Ph is different from an unsubstituted phenyl radical.
